# EUROPEAN PATENT APPLICATION

(11) **EP 2 573 071 A1**
(43) Date of publication of application: **27.03.2013**
(21) Application number: 10851630.3
(22) Date of filing: 09.09.2010
(51) Int. Cl.: C07D 207/273

(54) **PREPARATION METHOD OF (S)-4-HYDROXY-2-OXO-1-PYRROLIDINE ACETAMIDE**

(30) Priority: 21.05.2010 CN 201010179830
(71) Applicant: Chongqing Runze Medical Equipment And Supplies Co., Ltd, Yubei, Chongqing 401120 (CN)
(72) Inventor: CHEN, Yuying, Chongqing 401120 (CN); RONG, Zuyuan, Chongqing 401120 (CN); JIN, Lei, Chongqing 401120 (CN); FENG, Hua, Chongqing 401120 (CN); LI, Fei, Chongqing 401120 (CN); LI, Bo, Chongqing 401120 (CN); YOU, Chao, Chongqing 401120 (CN); PANG, Qi, Chongqing 401120 (CN); YE, Lei, Chongqing 401120 (CN); XU, Nan, Chongqing 401120 (CN)
(74) Representative: Chaillot, Geneviève
(86) International application number: PCT/CN2010/076758
(87) International publication number: WO 2011/143873

(57) **Abstract**

A preparation method of (S)-4-hydroxy-2-oxo-1-pyrrolidine acetamide is provided, which includes the steps of preparing the crude product and crystallizing, wherein acetone and water are used as solvents in the step of crystallizing. The (S)-oxiracetam prepared by the method of the present invention has high purity of more than 99.3% and low impurity of 0-0.5%, based on the percentages of the mass. According to the method of the present invention, with regard to the way of charging materials, adding inorganic base only just a few times is easier to handle and more convenient to the industrial manufacturing and application.

## Description

### BACKGROUND OF THE INVENTION

### 1. Field of Invention

The present invention relates to a method for preparing (S)-4-hydroxy-2-oxo-1-pyrrolidine acetamide.

### 2. Description of the Related Art

(S)-4-hydroxy-2-oxo-1-pyrrolidine acetamide is a levorotatory form of oxiracetam, also called commercially (S)-oxiracetam [hereinafter the (S)-oxiracetam]. The chemical structure of (S)-oxiracetam is shown below:

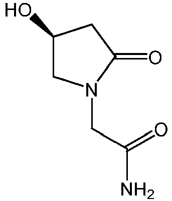

As reported, it is published that oxiracetam is a synthetic cyclic compound derivatives of -Amino- -hydroxybutyric acid (GABOB) is one of the efficacious drugs for Alzheimer's Disease (AD), vascular disease or the like, which can promote ATP in the brain and the synthesis of acetylcholine, and also enhance transmission of the nerve excitation, improve the retrograde amnesia resulting from lack of oxygen, enhance memory and strengthen learning ability. It is found in our researches that the efficacy described above of (S)-oxiracetam is much better than that of racemic oxiracetam. Some patents disclose preparation methods of levo-oxiracetam, such like WO/2005115978, CN101367757. CN101367757 discloses a (S)-4-hydroxy-2-oxo-1-pyrrolidine acetamide, which comprises steps of: (a) adding 518.4g glycinamide HCl, 394g sodium bicarbonate and 3.7L pure ethanol into three-neck RB flask, controlling the pH value being about 7.4, agitating with increasing the temperature and refluxing; (b) after 2-hour refluxing, adding 781.6g (S)-4-chloro-3-hydroxybutyryl acetate and the surplus 394g sodium bicarbonate in eight batches at the same time, and controlling the amount of the added base through measuring the pH value, to assure the pH value being equal to or less than 8.5 during the additions; (c) refluxing and reacting for 24 hours after adding (S)-4-halo-3-hydroxybutyryl acetate, ending the reaction until the amount of (S)-4-hydroxy-2-oxo-1-pyrrolidine acetamide reaches75% by measurement through HPLC, and obtaining a crude product of (S)-4-hydroxy-2-oxo-1-pyrrolidine acetamide through hot filtration and concentration; (d) dissolving the crude product in 800mL water, treating the solution with 001×7 strongly acidic styrene type cation exchange resin 8.0L, then collecting products, treating the products with 201×7 strongly basic styrene type cation exchange resin to neutralize the collected products, and ending the neutralization until the pH value reaches 7.0±0.1; (e) concentrating and drying the neutralized products, adding the dried product into 750 mL ethanol with heating, adding 10g charcoal to decolorize for 30 minutes after the dried product is dissolved, then filtering, cooling and crystallizing to obtain 240g (s)-oxiracetam in a white crystal form, and recrystallizing the white crystal with methanol/propanol (ratio of volume: 1/3) mixed solution (360m1/1080m1) to obtain 160g (S)-oxiracetam (HPLC: 99.3%).

However, the process described above is practically not useful for industrial manufacturing. Also, the produced (S)-oxiracetam has relatively high impurity. It is also found that (S)-oxiracetam has a variety of crystal forms depending on different preparing methods.

### SUMMARY OF THE INVENTION

The primary objective of the present invention is to provide a method for preparing (S)-4-hydroxy-2-oxo-1-pyrrolidine acetamide (hereinafter the "(S)-oxiracetam") with high purity and lower impurities therein.

To achieve the objective, a method for preparing (S)-oxiracetam in accordance with the present invention comprises steps of: preparing crude product and a post-treatment, wherein the post-treatment comprises the step of: crystallizing the crude product by using acetone and water as solvents.

The water and acetone are in a ratio ranging from 1:5 to 1:20, based on the weight parts.

The step of crystallizing the crude product is performed at low temperature, and more particularly at between -10°C and 10°C.

The step of crystallizing the crude product further comprises steps of: dissolving the crude product in water, adding acetone in drops at the temperature of between -10°C and 10°C, and agitating for 0.5 hours to 12 hours to obtain a crystalline product; the crude product and the water are in a ratio ranging from 1:0.4 to 1:0.7, the water and the acetone are in a ratio ranging from 1:5 to 1: 20, based on the weight parts.

The step of crystallizing the crude products is performed at room temperature, and further comprises the steps of: dissolving the crude product in water, filtering, and agitating with decreasing the temperature to a range of between -10°C and 10°C, then adding acetone in drops, agitating for 0.5 hours to 12 hours at the same temperature, filtering, and washing with cold acetone to obtain a crystalline product; the crude product and the water are in a ratio ranging from 1:0.4 to 1: 0.7, the water and the acetone are in a ratio ranging from 1:5 to 1: 20, based on the weight parts.

For further improvement of the purity of the final products for the preparation process, the post-treatment can comprises: refining the crude product before the step of crystallizing the crude product, and specifically, adding ethanol in a weight of 2 to 8 times larger than that of the crude product, then agitating and filtering to obtain a refined product. Alternatively, the post-treatment can also comprises: recrystallizing the crystalline product after the step of crystallizing the crude product.

For distinguishing the two crystalline products made by crystallization and recrystallization, hereinafter the crystalline product made by crystallization is called "the crystalline product", and the other made by recrystallization is called "the recrystalline product".

The step of recrystallizing the crystalline product is using acetone and water as solvents, and the water and acetone are in a ratio ranging from 1:5 to 1:20, based on the weight parts.

The step of recrystallizing the crystalline product is performed at low temperature, and more particularly at between -10°C and 10°C.

The step of recrystallizing the crystalline product further comprises steps of: dissolving the crystalline product in water, adding acetone in drops at the temperature of between -10°C and 10°C, and agitating for 0.5 hours to 12 hours to obtain a recrystalline product; the crystalline product and the water are in a ratio of from 1:0.4 to 1: 0.7, the water and the acetone are in a ratio of from 1:5 to 1: 20, based on the weight parts.

The step of recrystallizing the crystalline product is performed at room temperature, and further comprises steps of: dissolving the crystalline product in water, filtering, and agitating with decreasing the temperature in a range of between -10°C and 10°C, then adding acetone in drops, agitating for 0.5 hours to 12 hours at the same temperature, filtering, and washing with cold acetone to obtain a recrystalline product; the crystalline product and the water are in a ratio ranging from 1:0.4 to 1: 0.7, the water and the acetone are in a ratio ranging from 1:5 to 1: 20, based on the weight parts.

The post-treatment further comprises the step of: extracting, and the solvent used for extracting is methylene dichloride.

Specifically, the method for preparing (S)-oxiracetam comprises the steps of:
1. mixing glycinamide HCl, pure ethanol and partial or total sodium bicarbonate and agitating with increasing the temperature and refluxing to carry out the reaction;
2. after refluxing for 2 hours, optionally adding the surplus sodium bicarbonate, and adding (S)-4-chloro-3-hydroxybutyryl acetate in drops, then refluxing and reacting for 24 hours to form a solution, filtering the solution after it being cooled, and then concentrating the filtered solution to make an intermediate product; (S)-4-chloro-3-hydroxybutyryl acetate and glycinamide HCl are in a molar ratio of between 1:0.8 and 1:1.2, (S)-4-chloro-3-hydroxybutyryl acetate and the sodium bicarbonate are in a molar ratio of 1:2, and (S)-4-chloro-3-hydroxybutyryl acetate and the ethanol are in a mole-to-volume ratio of between 1mole:600 ml and 1mole:100ml;
3. dissolving the intermediate product in water and extracting four times by using methylene dichloride in a volume four times larger than that of the water used in dissolving the intermediate product to obtain a solvent extract, concentrating the solvent extract and removing the remaining methylene dichloride, diluting the concentrated solvent extract and passing it through the 001X7 cation exchange resin, collecting product fractions of the concentrated solvent extract, neutralizing the product fractions by cation exchange resin 201X7, then filtering the product fractions to remove the remaining resin therein, concentrating the product fractions and adding charcoal in the middle of the process, then agitating for 30 minutes, filtering and concentrating again to obtain a crude product;
4. crystallizing: adding water to the crude product at room temperature, then filtering, agitating with the decreasing temperature in a range of 2°C to 5°C, adding acetone in drops, keeping agitating for 30 minutes at the same temperature, then filtering and washing 2 or 3 times with cold acetone to obtain a crystalline product; the crude product and the water are in a ratio ranging from 1:0.4 to 1:0.7, and the water and the acetone are in a ratio ranging from 1:5 to 1:20, based on the weight parts;
5. recrystallization: dissolving the crystalline product in water at room temperature, then filtering and washing 2 or 3 times with cold acetone to make precipitants, keeping adding acetone, agitating for 30 minutes, then filtering and washing 2 or 3 times with cold acetone to obtain a recrystalline product; the water and the acetone are in a ratio ranging from 1:5 to 1:20, based on the weight parts.

The advantages of the present invention are described below:
The operation manner of the method of the present invention is simple and easy to handle. With regard to the way of charging materials, adding inorganic base only 1 time or 2 times is much convenient to the industrial manufacturing and application. According to the method of the present invention, using methylene dichloride to extract impurities in the aqueous phase and using acetone and water as solvents for crystallization/recrystallization greatly improve the quality of the final products. The (S)-oxiracetam produced by the method of the present invention has high purity of more than 99.3%, low impurities of about 0-0.5%, based on the percentages of the mass.

### DETAILED DESCRIPTION OF THE INVENTION

The present invention is more specifically described in the following paragraphs by way of examples. It is necessarily noted that the description, together with details of the structure and function of the invention is illustrative only and not limit the principles of the invention to the embodiments in the disclosure. People skilled in the art will recognize that the invention can be practiced with modification within the spirit and scope of the claims.

### Example 1:

The method for preparing (S)-oxiracetam comprises steps of:
1. charging 65.0g glycinamide HCl, 500ml pure ethanol and 49.3g sodium bicarbonate into three-necked round-bottom flask, and agitating with increasing the temperature and refluxing;
2. adding 49.3 sodium bicarbonate after refluxing for 2 hours, adding 97.7g (S)-4-chloro-3-hydroxybutyryl acetate in drops, then refluxing and reacting for 24 hours to form a solution, filtering the solution after it being cooled, and then concentrating the filtered solution to obtain a reddish brown oily substance;
3. dissolving the reddish brown oily substance in 65ml water and extracting four times with 260ml methylene dichloride (65ml methylene dichloride used per time) to obtain a solvent extract, concentrating the solvent extract and removing the remaining methylene dichloride, diluting the concentrated solvent extract and passing it through the 001X7 cation exchange resin, collecting the product fractions of the concentrated solvent extract, neutralizing the product fractions by cation exchange resin 201X7, then filtering the product fractions to remove the remaining resin therein, concentrating the product fractions and adding charcoal in the middle of the process, then agitating for 30 minutes, filtering and concentrating again to obtain a crude product;
4. crystallizing: adding water to the crude product at room temperature, then filtering, agitating with decreasing the temperature in a range of 2°C to 5°C, adding acetone in drops, keeping agitating for 30 minutes at the same temperature, then filtering and washing 2 times with cold acetone to obtain a crystalline product in an amount of 39.6g; the crude product and the water are in a weight ratio of ranging from 1:0.4 to 1:0.7, and the water and the acetone are in a ratio of ranging from 1:5 to 1:20, based on the weight parts;
5. recrystallizing: dissolving the crystalline product in water at room temperature, then filtering and washing the solution 2 or 3 times with cold acetone to make precipitants, keeping adding acetone until the volume of the added acetone is ten times larger than that of the water, agitating for 30 minutes, then filtering and washing 2 times with cold acetone to obtain a recrystalline product in an amount of 31.6g and having chromatographic purity: 99.64%, specific rotation: [α]_{D}²⁰ = -38.0(C=1.0 for water).

### Example 2

1. charging 65.0g glycinamide HCl, 500ml pure ethanol and 49.3g sodium bicarbonate into three-necked round-bottom flask, and agitating with increasing the temperature and refluxing;
2. adding 49.3 sodium bicarbonate after refluxing for 2 hours, adding 97.7g (S)-4-chloro-3-hydroxybutyryl acetate in drops, then refluxing and reacting for 24 hours to form a solution, filtering the solution after it being cooled, and then concentrating the filtered solution to obtain a reddish brown oily substance;
3. dissolving the reddish brown oily substance in 65ml water and extracting four times by using 260ml methylene dichloride (65ml methylene dichloride used per time) to obtain a solvent extract, concentrating the solvent extract and removing the remaining methylene dichloride, diluting the concentrated solvent extract and passing it through the 001X7 cation exchange resin, collecting the product fractions of the concentrated solvent extract, neutralizing the product fractions by cation exchange resin 201X7, then filtering the product fractions to remove the remaining resin therein, concentrating the product fractions and adding charcoal in the middle of the process, then agitating for 30 minutes; filtering and concentrating again to obtain a crude product;
4. refining: adding ethanol of a weight 3 times larger than that of the crude product, then agitating and filtering to obtain a refined crude product;
5. crystallizing: adding water to the refined crude product at room temperature, then filtering, agitating with the decreasing the temperature in a range of 2°C to 5°C, adding acetone in drops, keeping agitating for 30 minutes at the same temperature, filtering and then washing 2 times with cold acetone to obtain a crystalline product in an amount of 24.7g and having chromatographic purity: 99.35%.

### Example 3 to 6:

The materials and the amounts thereof used in Example 3 to 6 are shown in Table 1, while other factors and steps are all the same as described in Example 1. The produced (S)-oxiracetam has purity of higher than 99.3%, low impurities of about 0-0.5%, based on the percentages of the mass.

**Table 1**

| Example | (S)-4-chloro-3-hydroxybutyryl acetate | Glycinamide HCl | The amount of ethanol | The amount of sodium carbonate | Refining and the relative weight of ethanol of the crude product | The materials used in the crystallization (the ratio on the basis of weight parts) | The temperature of the crystallization; time of agitating | Recrystallization |
|---|---|---|---|---|---|---|---|---|
| 3 | 97.7g | 64.8g | 500ml | 98.5g | Not performed | crude product: water = 1: 0.4; water: acetone = 1: 20 | -10-5°C; 2h | Not performed |
| 4 | 78.2g | 51.8g | 400ml | 78.8g | Not performed | crude product: water =1: 0.7; water: acetone =1: 5 | 8-10°C; 12h | Same factors as those of the first crystallization |
| 5 | 156.4g | 103.6g | 800ml | 157.6g | Performed, the amount of ethanol is 8 times larger than the crude product | crude product: water =1 : 0.5; water : acetone =1 : 10 | 0-5°C; 5h | Not performed |
| 6 | 4.9Kg | 3.3Kg | 25L | 4.9Kg | Performed, the amount of ethanol is 2 times larger than the crude product | crude product: water =1 : 0.6; water : acetone =1 : 8 | 2-6°C; 3h | Not performed |

## Claims

1. A method for preparing (S)-4-hydroxy-2-oxo-1-pyrrolidine acetamide comprising steps of:
preparing crude product and a post-treatment, wherein the post-treatment comprises the step of: crystallizing the crude products using acetone and water as solvents.

2. The method as claimed in claim 1, wherein the water and acetone are in a ratio ranging from 1:5 to 1:20, based on the weight parts.

3. The method as claimed in claim 1 or 2, wherein the step of crystallizing the crude product is performed at low temperature.

4. The method as claimed in claim 3, wherein the step of crystallizing the crude product is performed at between -10°C and 10°C.

5. The method as claimed in claim 1, wherein the step of crystallizing the crude product further comprises the steps of: dissolving the crude product in water, adding acetone in drops at the temperature of between -10°C and 10°C, and agitating for 0.5 hours to 12 hours to obtain a crystalline product.

6. The method as claimed in claim 5, wherein the step of crystallizing the crude product is performed at room temperature, and further comprises the steps of: dissolving the crude product in water, filtering, and agitating with decreasing the temperature in a range of between -10°C and 10°C, then adding acetone in drops, agitating for 0.5 hours to 12 hours at the same temperature, filtering, and washing with cold acetone to obtaining a crystalline product; the crude product and the water are in a ratio ranging from 1:0.4 to 1:0.7, the water and the acetone are in a ratio ranging from 1:5 to 1: 20, based on the weight parts.

7. The method as claimed in claim 1, 2, 5 or 6, wherein the post-treatment further comprises the step of: refining the crude product with ethanol before the step of crystallizing the crude product, or recrystallizing the crystalline product after the step of crystallizing the crude product.

8. The method as claimed in claim 7, wherein the step of refining the crude product further comprises the steps of: adding ethanol in a weight of 2 to 8 times larger than that of the crude product, and agitating and filtering to obtain a refined product.

9. The method as claimed in claim 7, wherein the step of recrystallizing the crystalline product is using acetone and water as solvents, and the water and the acetone are in a ratio ranging from 1:5 to 1:20, based on the weight parts.

10. The method as claimed in claim 9, wherein the step of recrystallizing the crystalline product is performed at low temperature of between -10°C and 10°C.

11. The method as claimed in claim 7, wherein the step of recrystallizing the crystalline product further comprises the steps of: dissolving the crystalline product in water, adding acetone in drops at the temperature of between -10°C and 10°C, agitating for 0.5 hours to 12 hours to obtain a recrystalline product; the crystalline product and the water are in a ratio of from 1:0.4 to 1: 0.7, the water and the acetone are in a ratio of from 1:5 to 1:20, based on the weight parts.

12. The method as claimed in claim 1, 2, 5 or 6, wherein the post-treatment further comprises the step of: extracting, and the solvent used for extracting is methylene dichloride.

13. The method as claimed in claim 1, wherein it comprises steps of:
a. mixing glycinamide HCl, pure ethanol and partial or total sodium bicarbonate and agitating with increasing the temperature and refluxing to carry out the reaction;
b. after refluxing for 2 hours, optionally adding the surplus sodium bicarbonate, and adding (S)-4-chloro-3-hydroxybutyryl acetate in drops, then refluxing and reacting for 24 hours to form a solution, filtering the solution after it being cooled, and then concentrating the filtered solution to make an intermediate product, wherein (S)-4-chloro-3-hydroxybutyryl acetate and glycinamide HCl are in a molar ratio of between 1:0.8 and 1:1.2, (S)-4-chloro-3-hydroxybutyryl acetate and the sodium bicarbonate are in a molar ratio of 1:2, and (S)-4-chloro-3-hydroxybutyryl acetate and the ethanol are in a mole-to-volume ratio of between 1mole:600 ml and 1mole:100ml;
c. dissolving the intermediate product in water and extracting four times by using methylene dichloride in a volume four times larger than that of the water used in dissolving the intermediate product to obtain a solvent extract, concentrating the solvent extract and removing the remaining methylene dichloride, diluting the concentrated solvent extract and passing it through the 001X7 cation exchange resin, collecting product fractions of the concentrated solvent extract, neutralizing the product fractions by cation exchange resin 201X7, then filtering the product fractions to remove the remaining resin therein, concentrating the product fractions and adding charcoal in the middle of the process, then agitating for 30 minutes, filtering and concentrating again to obtain a crude product;
d. crystallizing: adding water to the crude product at room temperature, then filtering, agitating with the decreasing the temperature in a range of 2°C to 5°C, adding acetone in drops, keeping agitating for 30 minutes at the same temperature, then filtering and washing 2 or 3 times with cold acetone to obtain a crystalline product; the crude product and the water are in a weight ratio ranging from 1:0.4 to 1:0.7, and the water and the acetone are in a ratio ranging from 1:5 to 1:20, based on the weight parts;
e. recrystallizing: dissolving the crystalline product in water at room temperature, then filtering and washing 2 or 3 times with cold acetone to make precipitants, keeping adding acetone, agitating for 30 minutes, then filtering and washing 2 or 3 times with cold acetone to obtain a recrystalline product; the water and the acetone are in a ratio of ranging from 1:5 to 1:20, based on the weight parts.
